# EUROPEAN PATENT APPLICATION

(11) **EP 2 631 657 A1**
(43) Date of publication of application: **28.08.2013**
(21) Application number: 12001255.4
(22) Date of filing: 25.02.2012
(51) Int. Cl.: G01N 33/68

(54) **Immunoassay for the detection of the 22kDa C-terminal fragment (CAF) of agrin**

(71) Applicant: Neurotune AG, 8952 Schlieren (CH)
(72) Inventor: Dahinden, Pius, 5412 Gebenstorf (CH); Hettwer, Stefan, 5102 Rupperswil (CH); Vrijbloed, Jan Willem, 4313 Möhlin (CH)
(74) Representative: Emmel, Thomas

(57) **Abstract**

Immunoassay for the detection of CAF (22-Kd fragment of agrin) in a sample, which additionally can include larger agrin-fragments (110-Kd fragment) and agrin itself and in which the CAF to be detected can be present in one or both of the following 2 different conformations CAF-closed and CAF-open, wherein in an optional first step the larger fragments and agrin possibly present in the sample are removed therefrom, in a second step the remaining portion of the sample obtained after possible removal of the larger fragments in the first step is incubated with a (primary) antibody with known binding properties to the different conformations of the CAF, with the antibody being capable to specifically bind to at least the CAF-open conformation, and in a third step it is determined whether or not antibody-CAF-complexes have been formed after incubation.

SEQUENCE LISTING

<110> neurotune AG
<120> Immunoassay for the detection of the 22kDa C-terminal fragment
(CAF) of Agrin
<130> 05701ep
<160> 1
<170> PatentIn version 3.5

<210> 1
<211> 7
<212> PRT
<213> Artificial Sequence

<220>
<223> Epitope Sequence
<400> 1
Pro Val Thr Gly Ser Ser Pro
1               5

## Description

The invention relates to an immunoassay for especially the detection of a specific in vivo produced agrin-fragment, namely the 22kDa C-terminal fragment (CAF).

Agrin is an important protein which plays a pivotal role in the synapse-formation process by assisting formation and maintenance of the postsynaptic apparatus of developing neuromuscular junctions (NMJ's) (Bezakova and Ruegg, 2003). It could be shown that agrin deficient mice die at birth due to respiratory failure. This is caused by the fact that agrin is strictly required for the proper innervation of muscle fibres and that these mice are not able to build proper NMJ's.

Agrin is not only existent in neural or neuronal tissues but can also be found in periphery tissues like the lung and the kidney which indicates that agrin plays also a role in these organs.

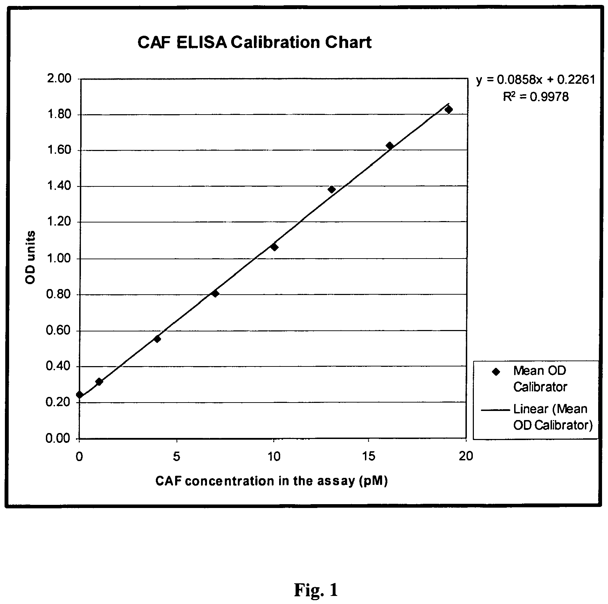

Agrin is a large heparan proteoglycan with a molecular weight of 400 - 600 kDa. (Database accession number NP_940978). The protein core consists of about 2000 amino acids and its mass is about 225 kDa. It is a multidomain protein composed of 9 K (Kunitz-type) domains, 2 LE (laminin-EGF-like) domains, one SEA (sperm protein, enterokinase and agrin) domain, 4 EG (epidermal growth factor-like) domains and 3 LG (laminin globular) domains (Fig. 1).

Agrin exists in several splice variants and can be expressed as a secreted protein, containing the N-terminal NtA (N-terminal agrin) domain, which is the most abundant form of agrin and the predominant form expressed in motor neurons. It is produced in the soma of the neurons, transported down the axon and released from the axon ending of the motor nerve into the synaptic cleft of the NMJ. Here it acts as an agonist of LRP4 (low-density lipoprotein receptor-related protein 4) and may also become a component of the basal lamina. In the CNS (central nervous system), most agrin is expressed as a type-II transmembrane protein by alternative splicing at the N-terminus lacking the N-terminal NtA domain (Bezakova and Ruegg, 2003).

Agrin is cleaved by an enzyme called neurotrypsin which plays an important role in controlling the activity of agrin. At present, agrin is the only known target of neurotrypsin. Neurotrypsin (Stephan A, et al., 2008) cleaves agrin at 2 distinct sites called alpha-and beta-site. (Fig. 1). The alpha-site is located N-terminal from the SEA domain and the beta-site is placed in front of the LG3 domain of agrin. Cleavage at the alpha-site generates a ∼110 kDa C-terminal agrin-fragment running at ∼130 kDa in a 4-12 % bis-tris SDS gel. Cleavage at the beta-site liberates the C-terminal LG3 domain running at ∼22 kDa in the gel (Molinari et al., 2002; Reif et al., 2007). Cleavage at the beta-site leads to a separation of the LG2 domain from the LG3 domain.

It was found that neurotrypsin (NT) over-expressing mice, so-called sarcopenia mice (muslik, M491 S) (Stephan et al., 2008), show an early onset of sarcopenia, a degenerative loss of skeletal muscle mass and strength associated with aging.

In the C-terminal part of human agrin, there are 2 alternative splice sites y and z. At the y-site, there may be inserts of 0, 4, 17 or 21 (4+17) amino acids and at the z site there may be inserts of 0, 8, 11 or 19 (8+11) amino acids. The function of the four inserted amino acids in the y-site is to create a heparin binding site. Motor neurons express predominantly y4 agrin. The most important splice site of agrin in respect of NMJ maturation is the z-site, giving agrin the ability to be active as an acetylcholine-receptor clustering agent. It is well known that full length agrin containing the insertion of 8 amino acids at the z-site in presence of the 4 amino acid insert in splice site y (y4z8) generates an agrin variant with a half maximal AChR clustering activity of 35 pM in cultured myotube clustering assays. The insertion of 11 amino acids give rise to a half maximal AChR clustering activity of 5 nM while the 19 amino acid insertion results in a half maximal AChR clustering activity of 110 pM. Agrin without an insertion at this site is not active in clustering acetylcholine-receptors on the in-vitro cultured myotubes (Bezakova and Ruegg, 2003). Thus, the most active form of agrin in the clustering assay is the y4z8 variant, which is expressed by motor neurons.

All C-terminal fragments could be detected in brain extracts and spinal cord extracts of mice (Stephan et al., 2008). In neurotrypsin knock out mice, none of the fragments could be detected. As a consequence, neurotrypsin seems to be the only protease which cleaves agrin in significant amounts at the two cleavage sites. Cleavage of agrin by neurotrypsin can generate in principle two different CAF containing agrin-fragments, besides full length agrin CAF and C110 are agrin-fragments that can be detected in blood. These fragments, also depending on the presence of specific inserts at the y and z position, can have different functions. CAF can be produced in two different ways, first as recombinantly produced 22-kd protein or after neurotrypsin cleavage of a large agrin-fragment. A list with the CAF containing naturally occurring agrin-fragments are described in the table 1 below.

**Table 1**

| Abbreviation | Description |
|---|---|
| C22, CAF | Naturally occurring 22kd C-terminal agrin-fragment generated by neurotrypsin cleavage of agrin at the beta cleavage site. The 22-kD corresponds to the apparent running position on PAGE gel. Insert at the Z position is not fixed. There can be no insert (CAF-z0) or inserts of 8 (CAF-z8), 11 (CAF-z11), or 19 (CAF-z19) amino acids, respectively. Species is not determined and could be from e.g. human-derived (human CAF), rat derived (rat CAF), mouse-derived (mouse CAF), chicken etc. For in-stance, human CAF-z8 represents the 22kd C-terminal human-derived agrin-fragment generated by neurotrypsin cleavage of agrin with an 8 amino acid insert at the Z position. |
| C110 | Naturally occurring C-terminal 110kd agrin-fragment gener-ated by neurotrypsin cleavage of agrin at the alpha site. The 110-kD size corresponds to the apparent running position on PAGE gel. Can have inserts at the y and z position. Species is not determined and could be from e.g. human, rat, mouse, chicken etc. For instance, human C110-y4z8 represents the human derived C-terminal 110 kd agrin-fragment having the 4 amino acid insert at the Y position and 8 amino insert at the Z position. |
| Ag | Full length agrin that is not cleaved at the alpha site or beta sites. Can have inserts at the y and z position. Species is not determined and could be from e.g. human, rat, mouse, chicken etc. For instance, human C110-y4z8 represents the human de-rived C-terminal 110 kd agrin-fragment having the 4 amino acid insert at the Y position and 8 amino insert at the Z posi-tion. |

### Description of different agrin-fragments

EP 1 990 420 by the same applicants as the present application, describes the use of the C-terminal 22-kDa agrin-fragment (CAF) of agrin as biomarker for the in vivo activity of neurotrypsin and in diagnosis and monitoring of neurotrypsin-related disturbances. The applicants found out that the presence and an elevated amount of this fragment which is liberated after cleavage of agrin at the beta-site and can be measured in body fluids such as serum correlates with certain disturbances like e.g. sarcopenia.

The presence of elevated CAF-levels could also be shown in patients suffering from dysfunctions of the kidney and the lung or suffering from diseases of the brain like autism or mental retardation. Patients suffering from mild cognitive impairment, early dementia or Alzheimer can have elevated levels of CAF in cerebrospinal fluid (CSF).

Alternatively, CAF levels can be reduced compared to normal healthy persons in patients suffering from neuropathic pain. It was found that peripheral nerve injury decreased agrin expression in the ipsilateral spinal dorsal horn of rats displaying tactile allodynia. Agrin modulates neuropathic pain through NR1 phosphorylation in GABA neurons. NR1 is part of the NMDA receptor, the NMDA receptor forms a heterotetramer between two NR1 and two NR2 subunits. The NMDA receptor, a glutamate receptor, is the predominant factor in diseases where there is a modulation of the NMDAR function: like Ischemia, Seizures Parkinson disease, Huntington's disease, Pain, Diabetes (peripheral NMDAR involved), Multiple Sclerosis, Schizophrenia, Autism, Alzheimer Diseases and other dementias or cognitive impairments molecular device for controlling synaptic plasticity and memory function.

Agrin reduction, and subsequent reduction in CAF levels may also open new approaches for detection of not only neuropathic pain, but also epilepsy, tremors, and spasticity (Cui and Bazan, 2010).

As was reported CAF inhibits the alpha 3 subunit of NaK-ATPase which could be a common triggering factor in the observed dysfunctions or diseases respectively.

From the above it appears that especially CAF but also other C-terminal fragments of agrin which can be detected in different tissues and correlated to different pathologic and non-pathologic conditions is not only an important marker but also an important target, which can be used in treatment of patients.

One problem, however, is connected to the measurement of CAF. In the EP 1 990 420 mentioned above it disclosed that CAF can be measured by Western-Blot assays which are able to detect CAF present in serum after denaturation with Guanidine. Western-Blot assays are not suitable for high-throughput proceedings.

The object of the invention is to provide an improved test for CAF especially a test in the Elisa-format which allows reliable detection and quantification of in vivo produced CAF.

### Summary of the invention:

The above object is accomplished by an immunoassay according to claim 1.

Crucial in the development of the new assay was the observation by the applicants that samples containing CAF showed different signals when analyzed by Western Blot technique compared to non-denaturing immunoassays (like e.g. the first tests made by the applicants in their development of an assay of the Sandwich-Elisa-format). Samples giving e.g. a strong CAF-signal in Western Blot assays produced no or only a weak signal in non-denaturating immuno-assays. From this observation the applicants followed and lateron confirmed that CAF, depending on the specific splice variant, and thereby on its origin, is present in two different conformations, which will be referred to in the following as CAF-open and CAF-closed, with both conformations having different immunogenic properties. As it appears, the accessibility at least of some of the epitopes on the CAF-protein depends on its conformation. Most of the epitopes are accessible in the CAF-open conformation, whereas the accessibility of the epitopes in the CAF-closed conformation can be critical, especially when it is required that 2 antibodies bind to CAF as is the case in Sandwich-Elisa assays.

This confirms on a hypothesis made by "Hoch et al 1994". The authors of the publication found out that an antibody generated against the extreme C-terminus of nonhuman agrin only recognized agrin-fragments including an insert at the splice site Z while in absence of such insert no binding occurred. From this they speculated that the fragment in question can be present in different conformations.

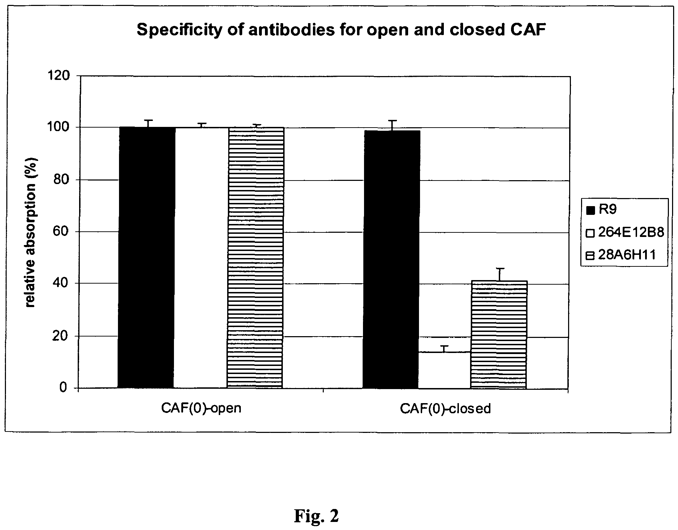

The consequence is that antibodies, which bind to the CAF-open conformation not necessarily bind to the CAF-closed conformation, which presents a high element of uncertainty in known immunoassays for undenaturated CAF and which may explain their poor performance (see Fig. 2)

As the applicants furthermore found out, the different conformations depend on the origin of the CAF. All recombinant forms of CAF, CAF(z0), CAF(z8), CAF(z11) and CAF(z19) are in the open conformation. Also neurotrypsin-produced CAF(z8), CAF(z11) and CAF(z19) are in the open conformation. Only CAF(0) is in the closed conformation and this is independent of neurotrypsin cleavage. So the CAF within agrin containing CAF(0) is in the closed conformation but also after neurotrypsin cleavage of agrin containing CAF(0) the CAF(z0) remains in the closed conformation.

As CAF(0) is the predominant form in vivo one can say that the usual conformation for CAF is the closed conformation.

From the above, it follows that a reliable immunoassay for CAF must take into account the different conformations in order to provide reliable results.

Accordingly, in the immunoassay according to claim 1, it is provided that an antibody is used for the detection of CAF, which at least detects the open-conformation. This means that the immunoassay according to the invention is performed with an antibody with known properties, i.e. it is known to the persons performing the assay that the antibody binds either to the CAF-open conformation alone or possibly also detects the CAF-closed conformation.

As a consequence if an immunoassay using antibodies which bind only to the open-conformation is negative, this does not necessarily mean, that no CAF is present in the sample, but it only allows the conclusion that the open-conformation is not present which does not exclude that the sample could include CAF in its closed conformation.

For the detection of the CAF closed conformation preferred embodiments of the invention are provided which are discussed below.

A second problem in CAF-assays is that especially after neurotrypsin cleavage of agrin there can be further larger fragments of agrin (110-Kd fragment) or agrin in its entity present in the sample. This is not relevant for urine or CSF but a problem in serum samples. The applicants have found out that it is essential to have these larger fragments and/or agrin molecules removed from at least serum samples before incubating the remaining sample with the antibody.

Summing up, the immunoassay according to the invention is the first one, which takes into account that different problems connected to CAF-detection and by doing so provides reliable results.

Preferred embodiments of the invention are addressed to in the sub claims. In one preferred embodiment it is provided that the immunoassay according to the invention is of the type-sandwich-ELISA.

In this context it can be provided that the primary antibody used is immobilized and that for marking of CAF-molecules possibly bound to the immobilized primary antibody a secondary antibody is used. Again it is essential that the binding properties of both antibodies are known with the provision that they bind at least the CAF-open conformation.

In a typical sandwich-ELISA 2 antibodies are used, a primary catcher antibody and a secondary detector antibody. Suitable antibody pairs can be selected from the antibodies listed below. When used in combination they recognize only CAF open and agrin or agrin-fragments that contain CAF-open. They were first described in EP 11000898 by the same applicants. Suitable mAB pairs are e.g. 264B12A8 with 28A6H11, 14B7B8, 28H7G3 or 28F7A6. Another suitable antibody pairs are 264E12B8 in combination with 28A6H11, 14B7B8, 28H7G3 or 28F7A6.

All antibodies mentioned above and the antibodies 14C5G4 and 12A11D11 mentioned later have been deposited in connection with EP11000898 on January 11, 2011 at DSMZ - Deutsche Sammlung von Mikroorganismen und Zellkulturen GmbH, Inhoffenstraße 7 B, 38124 Braunschweig GERMANY under the following accession numbers:

| | | | | | |
|---|---|---|---|---|---|
| 28H7G3 | = | DSMACC3101; | 264E12B8 | = | DSMACC3102 |
| 28A6H11 | = | DSMACC3103; | 14C5G4 | = | DSMACC3104 |
| 12A11D11 | = | DSMACC3105; | 28F7A6 | = | DSMACC3106 |
| 14B7B8 | = | DSMACC3107; | 264B12A8 | = | DSMACC3108 |

Further embodiments are directed to an immunoassay, which allows the detection of CAF-total, i.e. the detection of CAF present in its open and as well in its closed conformation in the sample.

A first embodiment in this context provides that the antibody used is capable to detect CAF in both conformations. As the applicants found out, each of the antibodies mentioned above is able to bind also to either the CAF-open or close-conformation which allows the design of assays of the Competition-Elisa type, i.e. immunoassays, in which only one antibody is used.

However, the development of assays for CAF-total from the Sandwich-Elisa type using the above antibodies was not yet successful. The reason most probably is, that the epitopes of these antibodies are to close together in the CAF-closed conformation, so that the simultaneous binding of 2 of these antibodies to CAF is not possible due to sterical hindrance.

A further preferred embodiment therefore is directed to an immunoassay in the sandwich-ELISA-format using a pair of antibodies which can bind simultaneously to CAF in both of its conformations.

Crucial was that the applicants isolated a polyclonal antibody, named R9 (see Fig. 2) which binds to an epitope more distant to the epitopes of the above antibodies. First results of the applicants show, that this antibody R9 binds to CAF without disturbing the binding of a further antibody as e.g. mentioned above. It is conceivable that there are further antibodies with similar properties. Using a pair of antibodies comprising R9 or a further antibody having a suitable epitope and e.g. one of the above mentioned antibodies it is possible to design an assay of the Sandwich-Elisa type for CAF-total.

The epitope sequence of R9 is PVTGSSP (SEQ ID NO.: 1). One can assume that the epitope sequence is well accessible in both conformations and in view of that a further embodiment of the invention provides monoclonal or polyclonal antibodies which recognise this epitope.

An alternative embodiment provides that prior to incubation with an antibody the sample is heated up to a temperature between 45 and 60 C° for a defined period of time between 5 and 120 minutes.

Preferably incubation is performed at a temperature between 50 and 58 C° for a period of time between 15 and 40 minutes, more preferably at a temperature between 55 and 57 degrees for a period of 25 and 35 minutes.

It has surprisingly turned out that by heating up the sample the CAF present therein in closed conformation will be converted to the open conformation. After conversion to the open conformation these CAF-molecules can be easily detected by antibodies, which bind to the open conformation. This embodiment allows the design of an assay of the sandwich-Elisa type as described in the example by using the pairs of antibodies mentioned above, i.e the antibodies which were submitted and described in connection with EP 11000898.

Applicants have found out that agrin could still be cleaved by neurotrypsin after the above defined heat treatment, which means that at least agrin but most probably its fragments are not denatured by the treatment.

As stated above, a further aspect of the invention is that larger agrin-fragments possibly present in a sample must be removed from the sample before its incubation with an antibody. Such larger fragments are always present in serum samples and for such samples the pretreatment is mandatory. Urine or CSF samples in contrast as a rule do not contain larger fragments and these samples can be assessed without pretreatment.

A preferred embodiment in this context provides that removal of the larger fragments and/or a whole agrin-molecules is done by incubating the sample with an antibody, which specifically detects this larger agrin-fragments and/or agrin and which do not bind to CAF. Suitable antibodies in this context can be the antibodies 14C5G4 and 12A11D11 described in EP 11000898. Both antibodies bind to sites located on the other N-terminal portion of the agrin-Fragment C44 described in the EP 11000898, i.e. they bind to a portion different from CAF.

Preferably, the antibodies used for removal are immobilized, so that an easy separation and removal from the remaining sample used for further processing is possible.

In a further preferred embodiment it is provided that the larger fragments and/or agrin-molecules are precipitated by either choosing special conditions and/or adding an agent, which alter the solubility of the sample. Examples for such conditions are a selection of a special pH-value or the addition of PEG. After treatment of the sample the precipitated larger fragments/agrin-molecules can be easily removed from the sample by centrifugation.

As stated above the immunassay according to the invention is the first one which takes into account that CAF present in a sample can have different immunogenic properties. In general one can say that CAF(0) is predominant in metabolism so that usual conformation on CAF will be the closed conformation. First measurements made by the applicants showed that the CAF-open concentration in healthy patients is in the range of 2-5 pM while the concentration of CAF-total (CAFopen + CAFclosed) ranges between 20-60pM. In contrast the CAF-open concentration in patients suffering from sarcopaenia amounts to about 10pM while the CAF-total concentration lies above 100pM. Patients suffering from kidney (renal failure) diseases have a CAF-open value above 25pM while the CAF-total concentration is above 300pM. The above values are not yet confirmed by extensive studies but are the results of first experiments and hence are only suited to give an impression of the absolute values for CAF-open and CAF-total an their ratios in different patients. What one can say already now is that the CAF-open concentration is different depending on the health situation of a patient. In a number of diseases determination of CAF-open will be sufficient, however, doubting other diseases determination of CAF-total is more suitable to make or strengthen a diagnosis.

From the present knowledge about the universal role of agrin it is evident that CAF is produced/present in a number of different tissues. Furthermore one can assume that CAF occurs in different splice variants in different tissues and additionally it is highly probable that also the conformation of CAF depends on its origin. In this context first results of the applicants discussed above indicate that the CAF-open to CAF-total concentrations in patients suffering of renal diseases are different from the corresponding ratio in patients suffering from sarcopenia or from those in healthy patients.

Consequently a final aspect of the invention is directed to the use of the test according to the invention to specifically determining absolute values of the different conformations of CAF and in a further preferred embodiment calculating the ratios of CAF open or CAF closed to CAF total of CAF closed to CAF open.

In the following the invention will be described by means of an example and figures.
- Fig. 1: shows a CAF-calibration curve used in the example 1.
- Fig. 2: shows the specificity of different antibodies for CAF in its closed and open conformation.

### EXAMPLE 1: Sandwich Elisa for CAF-total

### i) Microtiterplates

Microtiterplates (Maxi Sorp) coated with 125 µl per well with a solution of 0.5 µg/ml 264E12B8 or 264B12A8 in Coating buffer (12 mM Potassium-phosphate Buffer, pH 7,4-pH 8,2; 137 mM NaCl; 2.7 mM KCl) are used.

### ii) Sample preparation

For the assay undiluted serum samples are thawed at 37°C for 10 minutes. The samples are mixed and centrifuged for two minutes at 14000x g. In order to prepare samples for 1 ELISA plate, 50 µl Sample incubation buffer (50 mM potassium-phosphate buffer, pH 7,2-8,2; 137 mM NaCl; 2.7 mM KCl; 5 mg/ml HSA; 0-20 % Glycerol; 0-50% (NH4)2SO4 and or 10-30 % PEG 6000; 0.2-1 % Tween 20) are added to the wells of a protein LoBind deep well plate. Subsequently 50 µl serum sample are added to the same well and mixes with the incubation buffer by pipetting up and down 5-7 times. Additionally 50µl of calibrator solution are added to some of the wells of the plate and treated in the same way as the sample. The preparation of calibrator is explained below.

The LoBind plate is tightly sealed with an adhesive cover foil and incubated exactly 30 min at 56 °C in a waterbath. The plate will float on the water surface, shaking is not desirable at this stage. After incubation a precipitate is generated and in order to pellet the precipitate, the plate is centrifuged for 5 min at 3000 x g at room temperature. The cleared supernatant is diluted a tenfold with Sample dilution buffer (50 mM Potassium-phosphate buffer pH 7,2-8,2; 137 mM NaCl; 2.7 mM KCl) by pipeting 90 µl Sample dilution buffer in the wells of the pre coated ELISA plate (MTP). 10 µl of the cleared supernatant from the LoBind deepwell plate are carefully transferred (without disturbing the pellet) into the precoated ELISA plate. The samples are mixed by pipeting up and down a five to seven times. Then the MTP is tightly covered with an adhesive cover foil and incubated at room temperature (15-25 °C) for 16 h (over night)

### iii) Calibrator preparation

A thousand fold dilution of the CAF protein calibrator solution recombinant HsAgrC110_0.0 solution is prepared by making ten fold serial dilutions. The solution is diluted with Sample dilution buffer (50 mM Potassium-phosphate buffer pH 7,2-8,2; 137 mM NaCl; 2.7 mM KCl) to a final concentration of 400 pM. This stock solution serves as source in the preparation of the 8 calibrator series.

To prepare the calibrator series for the assay, the volumes of the 400 pM CAF stock solution and Sample dilution buffer (as indicated in table 2) are pipetted into 1.5 ml protein LoBind tubes. Mix by flicking the tubes several times. As stated above 50µl of the calibration series are added to the wells of the MTP and are then treated like the samples.

**Table 2**

| **calibrator concentration** | **400 pM CAF protein calibrator** | **Sample dilution buffer** |
|---|---|---|
| **0 pM** | **0 µl** | **200 µl** |
| **20 pM** | **10 µl** | **190 µl** |
| **80 pM** | **40 µl** | **160 µl** |
| **140 pM** | **70 µl** | **130 µl** |
| **200 pM** | **100 µl** | **100 µl** |
| **260 pM** | **130 µl** | **70 µl** |
| **320 pM** | **160 µl** | **40 µl** |
| **380 pM** | **190 µl** | **10 µl** |

### iv) ELISA protocol

All further incubation steps on the plate (MTP) are carried out at room temperature 15-25°C in the dark. Following the over night incubation (see ii) the wells of the MTP are washed 3 times with 300 µl Washing buffer (12 mM Potassium-phosphat Puffer pH 7,2-8,2; 137 mM NaCl; 2.7 mM KCl; 0-2% Casein; 0-0.5% Tween 20). Between each washing step the MTP is incubated 1 min at room temperature. The washing fluid is then removed by aspirating or tapping. After the wash steps are completed, 100 µl CAF detector antibody solution, 28A6H11-biotin or 28H7G3-Biotin or 14B7B8-biotin Conjugate (0.01-2 mg/ml) are pipetted in all wells. The MTP is then incubated room temperature for 30 min.

Subsequently the MTP is washed 3 times with washing buffer as described above and the washing fluid is removed by aspirating or tapping. Then 100 µl Strepta-vidin-polyHRP solution is pipetted in all wells and the MTP is incubated at room temperature for 30 min in the dark. The MTP is again washed 3 times with washing buffer as described above and the washing fluid is removed by aspirating or tapping. Then 100 µl TMB solution are pipetted in all wells and incubated at room temperature for 30 min in the dark. Finally, 100 µl Stop solution (Stop Reagent for TMB Microwell Substrates from SurModics) are pipetted in all wells and the optical density of each well at 450nm is measured on a microplate reader. The yellow color is stable for 1 hour at room temperature.

### v) Results

### a) Calibrator series

The calibrator solutions prepared as discussed above were used to prepare a seven point calibration curve. The resulting calibrator curve is shown in Fig.1. As can be seen from Fig. 1 the concentration of CAF and signal are in linear relationship.

### b) Serum samples

Serum from 20 healthy volunteers, sarcopenia patients and patients with renal failure were measured according the above described procedure. The serum samples were incubated at room temperature and at 56°C in order to measure CAF open and total CAF respectively. The results are depicted in table 3.

**Table 3**

| | Open CAF (pM) | Total CAF (pm) | % |
|---|---|---|---|
| Healthy volunteers | 3.40 | 41.8 | 8.1 |
| Sarcopenia patients | 11.3 | 124.3 | 9.0 |
| Patients with renal failure | 26.7 | 383.1 | 7.0 |

The results show as discussed above a clear relation between absolute CAF-open and CAF-total values with different states of health.

### EXAMPLE 2: Specificity of different antibodies for CAF in its closed and open conformation

A Nunc maxisorp microtiter plate was coated with 100 ul of a 2 ug/ml solution CAF(0)-closed and CAF(0)-open in coating buffer. The plates were incubated at 4 °C over night. Afterwards, the plates were washed 3 times with 300 ul washing buffer and blocked with 180 ul blocking solution (Candor Bioscience GmbH) for 3 h at room temperature. After 3 times washing with 300 ul washing buffer 100 ul of biotinylated antibodies R9, 264E12B8 or 28A6H11 at a concentration of 312 pg/ml in washing buffer were added and incubated for 30 min at room temperature. After a 3 times washing with 300 ul washing buffer 100 ul of a 62.5 ng/ml Streptavidine poly-HRP solution (PIERCE) in washing buffer were added and incubated for 30 min at room temperature. After 3 times washing with 300 ul washing buffer, 100 ul TMB substrate (Surmodix) was added and incubated at room temperature for 30 min. The reaction was stopped with Stop solution (Surmodix) and the plate was recorded at 450 nm in a Tecan Infinite F200 plate reader.

### The results are shown in Fig. 2

As can be seen from the Fig. 2 the polyclonal antibody R9 recognizes the open and closed forms of CAF equally well. The mABs 264E12B8, and 28A6H11 recognize the open form of CAF much better than the closed form.

### Reference List:

Bezakova G, Ruegg MA. New insights into the roles of agrin. Nat Rev Mol Cell Biol 2003 April;4(4):295-308.
Cui JG, Bazan NG. Agrin downregulation induced by nerve injury contributes to neuropathic pain. J Neurosci 2010 November 10;30(45):15286-97.
Hoch W, Campanelli JT, Harrison S, Scheller RH. Structural domains of agrin required for clustering of nicotinic acetylcholine receptors. EMBO J 1994 June 15;13(12):2814-21.
Molinari F, Rio M, Meskenaite V et al. Truncating neurotrypsin mutation in autosomal recessive nonsyndromic mental retardation. Science 2002 November 29;298(5599):1779-81.
Reif R, Sales S, Hettwer S et al. Specific cleavage of agrin by neurotrypsin, a synaptic protease linked to mental retardation. FASEB J 2007 November;21(13):3468-78.
Stephan A, Mateos JM, Kozlov SV et al. Neurotrypsin cleaves agrin locally at the synapse. FASEB J 2008 January 29;22(6):1861-73.

## Claims

1. Immunoassay for the detection of CAF (22-Kd fragment of agrin) in a sample, which additionally can include larger agrin-fragments (110-Kd fragment) and agrin itself and in which the CAF to be detected can be present in one or both of the following 2 different conformations CAF-closed and CAF-open, wherein
- in an optional first step the larger fragments and agrin possibly present in the sample are removed therefrom,
- in a second step the remaining portion of the sample obtained after possible removal of the larger fragments in the first step is incubated with a (primary) antibody with known binding properties to the different conformations of the CAF, with the antibody being capable to specifically bind to at least the CAF-open conformation, and
- in a third step it is determined whether or not antibody-CAF-complexes have been formed after incubation.

2. Assay according to claim 1, wherein the removal of larger agrin-fragments in step 1 is done by precipitation or by elimination with a specific antibody.

3. Assay according to claim 1, wherein determination whether or not antibody-CAF-complexes have been formed is made using a further (secondary) antibody being capable to specifically bind to at least the CAF-open conformation.

4. Assay according to claim 1, wherein prior to incubation the sample is heated up to a temperature of ca 45°-60C° for a defined period of time 5-120 minutes.

5. Assay according to claim 1, wherein the primary and/or secondary antibody are capable to detect CAF in both conformations.

6. Assay according to claim 4, wherein an antibody used recognises an epitope having the sequence PVTGSSP (SEQ ID NO: 1).

7. Use of the immunoassay according to claims 1-6 to determine the concentration of CAF closed or CAF open or CAF total initially present in the sample.

8. Use according to claim 7 wherein the ratio of CAF open or CAF closed to CAF total is calculated from the concentrations determined.
